# FASCICULE DE BREVET EUROPEEN

(11) **EP 0 714 653 B1**
(45) Date de publication et mention de la délivrance du brevet: **18.06.1997**
(21) Numéro de dépôt: 95402530.0
(22) Date de dépôt: 10.11.1995
(51) Int. Cl.: A61K 7/043, A61K 7/04

(54) **Composition translucide applicable sur l'ongle**
Durchscheinende Zusammensetzung zur Nagelpflege
Translucent nail composition

(30) Priorité: 02.12.1994 FR 9414537
(43) Date de publication de la demande: 05.06.1996
(73) Titulaire: L'OREAL, 75008 Paris (FR)
(72) Inventeur: Ramin, Roland, F-91760 Itteville (FR)
(74) Mandataire: Dodin, Catherine

(56) Documents cités:
- EP-A- 0 300 234
- EP-A- 0 453 628
- EP-A- 0 504 754
- FR-A- 1 453 089
- S.T.N., Serveur de bases de données, & ES-A-2 023 603 (HEREDIA ET AL.)
- PARFUMS, COSMETIQUES, AROMES, vol. 79, Mars 1988 PARIS, page 47-61 DJELASII ET AL. 'L'ongle: griffe ou ornement'

## Description

L'invention se rapporte à une composition applicable sur l'ongle naturel ou synthétique comprenant de la silice ainsi qu'à l'utilisation de silice dans une telle composition.

Les compositions applicables sur l'ongle, de type vernis à ongles, comprennent généralement comme base au moins un agent filmogène, au moins un plastifiant et au moins un solvant.
Ces compositions sont généralement très fluides et ne permettent pas un étalement correct de ladite composition sur l'ongle. En effet, la composition n'est pas déposée sur l'ongle en quantité suffisante. L'ajout d'au moins un composé choisi parmi les argiles, telle que la bentonite, permet d'épaissir la composition pour permettre un meilleur étalement sur l'ongle.
Toutefois, l'ajout d'un tel composé opacifie la composition tout en lui donnant une couleur plus ou moins jaunâtre désagréable pour l'oeil, au sein du flacon qui la contient. Cet aspect est généralement masqué par la présence de pigments et/ou de colorants dans la composition de vernis à ongles.
Il n'est donc pas possible de préparer une composition applicable sur l'ongle se présentant sous forme translucide lorsqu'elle contient un composé tel que la bentone.

Il est connu de la demande ES-A-2 023 603 un gel photodurcissable comprenant une résine polyester, de l'acide méthacrylique, de la silice pyrogénée comme agent thixotrope, un accélérateur de séchage et un photoinitiateur.

Une compsition de vernis à ongles comprenant un adhésif filmogène, une résine, un agent gélifiant, un plastifiant, des fibres courtes et un solvant volatil a été décrite dans le brevet FR-C-1 453 089.

Aussi le but de l'invention est de proposer une composition applicable correctement et en quantité suffisante sur l'ongle, qui ne soit pas opacifiante et jaunâtre dans le flacon qui la contient, tout en étant thixotrope c'est-à-dire pouvant passer d'un état gélifié à un état liquide par simple agitation, et inversement après repos.

Un objet de l'invention est donc une composition applicable sur l'ongle se présentant sous forme translucide, comprenant au moins un agent filmogène, au moins un agent plastifiant, au moins un solvant et de la silice pyrogénée.

La composition selon l'invention présente l'avantage de permettre l'obtention d'un film transparent sur l'ongle.
Un autre avantage de la composition selon l'invention est de se présenter sous forme légèrement gélifiée, ce qui permet l'incorporation de fines particules insolubles qui restent en suspension dans le milieu, sans modifier de manière significative la texture et la viscosité de la composition.

On peut ainsi obtenir une composition thixotrope et translucide qui comprend des particules en suspension.

L'invention a également pour objet l'utilisation de silice pyrogénée comme agent épaississant d'une composition translucide, éventuellement telle que définie précédemment.

L'invention se rapporte à une composition applicable sur l'ongle comprenant, comme base, au moins un agent filmogène, au moins un agent plastifiant et au moins un solvant. Elle comprend de plus de la silice pyrogénée.
L'agent filmogène présent dans la composition selon l'invention peut être choisi parmi la nitrocellulose ou une résine telle qu'une résine alkyde, acrylique, polyuréthanne, ou résultant de la condensation de formaldéhyde avec une arylsulfonamide.
L'agent filmogène peut être présent en une quantité de 5 à 20 %, de préférence 10-14 %, en poids par rapport au poids total de la composition.

L'agent plastifiant présent dans la composition selon l'invention peut être choisi parmi le camphre, les phtalates et/ou les esters.
L'agent plastifiant peut être présent en une quantité de 6 à 25 %, de préférence 16-20 %, en poids par rapport au poids total de la composition.

Le solvant présent dans la composition selon l'invention peut être choisi parmi les alcools éthylique, isopropylique, butylique, les acétates d'éthyle, de butyle, les cétones.
Le solvant peut être présent en une quantité allant de 60 à 85 % en poids, de préférence 72-78% en poids par rapport au poids total de la composition.
La composition selon l'invention peut également comprendre au moins un diluant qui peut être choisi parmi les hydrocarbures aromatiques ou aliphatiques tels que le toluène.
Le diluant présent dans la composition peut être présent en une quantité allant de 0 à 35% en poids, de préférence 20-27 % en poids, par rapport au poids total de la composition.

La silice présente dans la composition selon l'invention peut se présenter sous forme de silice pyrogénée hydrophile ou hydrophobe.
Les silices pyrogénées sont obtenues par hydrolyse à haute température d'un composé volatil de silicium dans une flamme oxhydrique. Il s'ensuit la production d'une silice finement divisée. Par réaction, on peut modifier chimiquement la surface, par réduction du nombre de groupes silanol afin d'obtenir une silice hydrophobe.
La silice peut être présente en une quantité allant de 0,1 à 3 % en poids, de préférence 0,4-0,8 % en poids par rapport au poids total de la composition.

La composition selon l'invention se présente donc sous forme translucide, c'est-à-dire sous une forme non opaque et non opalescente, ou encore sous une forme permettant le passage de la lumière sans permettre de distinguer les formes. Sa densité optique, mesurée dans une cuve en quartz de 10 mm, est de préférence inférieure à 1, préférentiellement de l'ordre de 0,1-0,6.

La composition selon l'invention peut comprendre, de plus, au moins un compose sous forme particulaire ou sous forme de fibres.
Ce composé sous forme particulaire ou sous forme de fibre peut être présent en une quantité allant de 0 à 2 % en poids par rapport au poids total de la composition, de préférence en une quantité comprise entre 0,01 et 0,5%.
Le composé sous forme de fibres peut être choisi parmi les fibres de Nylon et les fibres d'aramide.
Par l'expression "fibres d'aramide", on doit entendre des fibres de poly(paraphénylène-téréphtalamide) obtenues par polymérisation de la paraphénylènediamine et du chlorure de téréphtallyle. Parmi les fibres d'aramide qui peuvent être utilisées dans les compositions selon l'invention, on peut citer les fibres connues sous les dénominations commerciales de 'KEVLAR ®' en particulier de 'KEVLAR DRY PULP ®' de la société DUPONT DE NEMOURS, et 'ARENKA ®' de la société ENKA GLANZSTOFF.
Le composé sous forme de particules peut être choisi parmi les particules de diamant, les particules de nitrure de bore, les particules de nacre et/ou les particules de pierre ponce.
Le composé sous forme de particules peut avoir, de préférence, des dimensions de particules comprises entre 1 et 100 µm.

La composition selon l'invention peut comprendre de plus au moins un composé cosmétiquement actif, pouvant être choisi parmi les vitamines, les hydratants, les agents durcissant, les filtres solaires, les épaississants et/ou les matières premières d'origine biologique. La composition selon l'invention peut aussi comprendre au moins un colorant, voire un pigment minéral ou organique en petite quantité. Ces additifs peuvent être présents en une quantité allant de 0 à 3 % en poids par rapport au poids total de la composition.

La composition peut se présenter sous forme d'un vernis à ongles, très peu coloré, et/ou sous forme d'un produit de soin pour ongles.
Selon sa destination finale, elle peut comprendre les constituants habituellement présents dans de telles compositions.

L'invention a également pour objet l'utilisation de silice pyrogénée comme agent épaississant d'une composition se présentant sous forme translucide, notamment d'une composition à appliquer sur l'ongle.
On a en effet constaté que la composition selon l'invention se présente sous épaissie, voire gélifiée, par rapport à une composition ne comprenant pas de silice pyrogénée.

L'invention va maintenant être décrite plus en détail au moyen des exemples qui suivent et qui sont donnés uniquement à titre illustratif, et dans lesquels les pourcentages sont en poids.

### Exemple 1

| | |
|---|---|
| - Nitrocellulose | 10 g |
| - Plastifiant et résine | 15 g |
| - Acétate d'éthyle | 74,5 g |
| - Silice pyrogénée (Aerosil 200 ® hydrophile de Degussa) | 0,5 g |

La composition obtenue est translucide et de texture adéquate. Elle s'étale aisément sur les ongles et permet l'obtention d'un film uniforme et brillant adéquat.

### Exemple 2

| | |
|---|---|
| - Nitrocellulose | 10 g |
| - Plastifiant et résine | 15 g |
| - Acétate d'éthyle | 74,45 g |
| - Silice pyrogénée (Aerosil R972 ® hydrophobe de Degussa) | 0,5 g |
| - Poudre de diamant | 0,05 g |

La composition obtenue est translucide et de texture adéquate. Elle contient des particules de diamant qui restent en suspension. Elle s'étale aisément sur les ongles et permet l'obtention d'un film uniforme adéquat, qui présente une certaine brillance.

### Exemple 3

| | |
|---|---|
| - Nitrocellulose | 16 g |
| - Plastifiant et résine | 15 g |
| - Acétate d'éthyle | 67,9 g |
| - Silice pyrogénée | 0,8 g |
| - Particules de nacre | 0,3 g |

Cette composition est translucide et permet l'obtention d'un film uniforme possédant de bonnes propriétés cosmétiques.

### Exemple 4

On effectue une mesure de transmission directe de la lumière, sur plusieurs compositions comprenant de la silice pyrogénée (Aerosil 200®) ou de la bentone.
La composition comprend par ailleurs 10 g de nitrocellulose, 15 g de résine et de plastifiant et le complément à 100 g d'acétate d'éthyle.

On mesure la densité optique de ces compositions, d'une manière usuelle, en utilisant des cuves en quarts de 10 mm d'épaisseur.

On obtient les résultats suivants :

| Composition | Silice | Bentone | Densité optique |
|---|---|---|---|
| A | --- | 3 % | 2,250 |
| B | --- | 1% | 2,002 |
| C | --- | 0,5% | 1,272 |
| D | 3% | --- | 0,530 |
| E | 1% | --- | 0,253 |
| F | 0,5% | --- | 0,158 |
| Témoin | --- | --- | 0,026 |

On constate donc que les compositions comprenant de la silice présentent une densité optique faible, en comparaison avec les compositions comprenant un agent rhéologique usuel, la bentone.

De plus, une appréciation visuelle des différentes compositions donne les résultats qualitatifs suivants :
. compositions A, B et C : opaques, très diffusantes et d'aspect visqueux
. compositions D, E et F : translucides, assez fluides.

## Revendications

1. Composition applicable sur l'ongle se présentant sous forme translucide, comprenant au moins un agent filmogène, au moins un agent plastifiant, au moins un solvant, caractérisée en ce qu'elle comprend de plus de la silice pyrogénée.

2. Composition selon la revendication 1, caractérisée en ce que la silice est de la silice pyrogénée hydrophile ou hydrophobe.

3. Composition selon l'une des revendications précédentes, caractérisée en ce qu'elle comprend en outre au moins un composé sous forme particulaire et/ou sous forme de fibres.

4. Composition selon la revendication 3, caractérisée en ce que ledit composé est choisi parmi les fibres de Nylon, les fibres d'aramide, les particules de diamant, les particules de nitrure de bore, les particules de nacre et/ou les particules de pierre ponce.

5. Composition selon l'une des revendications précédentes, caractérisée en qu'elle comprend en outre au moins un composé cosmétiquement actif.

6. Composition selon la revendication 5, caractérisée en ce que le composé actif est choisi parmi les hydratants, les agents durcissants, les filtres solaires, les vitamines, les épaississants et/ou les matières premières d'origine biologique.

7. Composition selon l'une des revendications précédentes, caractérisée en ce que la silice est présente en une quantité allant de 0,1 à 3 %, de préférence 0,4-0,8% en poids, par rapport au poids total de la composition.

8. Composition selon l'une des revendications précédentes, caractérisée par le fait qu'elle présente une densité optique inférieure à 1, de préférence comprise entre 0,1 et 0,6.

9. Composition selon l'une des revendications précédentes, caractérisée en ce qu'elle se présente sous la forme d'un vernis à ongle.

10. Composition selon l'une des revendications précédentes, caractérisée en ce qu'elle se présente sous forme d'un produit de soin pour ongles.

11. Utilisation de silice pyrogénée comme agent épaississant d'une composition selon l'une des revendications 1 à 10.

## Claims

1. Composition applicable to the nail which is presented in translucent form, comprising at least one film-forming agent, at least one plasticizing agent, at least one solvent, characterized in that it comprises, in addition, pyrogenic silica.

2. Composition according to Claim 1, characterized in that the silica is hydrophilic or hydrophobic pyrogenic silica.

3. Composition according to either of the preceding claims, characterized in that it comprises, in addition, at least one compound in particulate form and/or in the form of fibres.

4. Composition according to Claim 3, characterized in that the said compound is chosen from Nylon fibres, aramid fibres, diamond particles, boron nitride particles, pearl particles and/or pumice particles.

5. Composition according to one of the preceding claims, characterized in that it comprises, in addition, at least one cosmetically active compound.

6. Composition according to Claim 5, characterized in that the active compound is chosen from moisturizers, hardening agents, sunscreens, vitamins, thickeners and/or raw materials of biological origin.

7. Composition according to one of the preceding claims, characterized in that the silica is present in a quantity ranging from 0.1 to 3%, preferably 0.4-0.8%, by weight relative to the total weight of the composition.

8. Composition according to one of the preceding claims, characterized in that it has an optical density of less than 1, preferably of between 0.1 and 0.6.

9. Composition according to one of the preceding claims, characterized in that it is presented in the form of a nail varnish.

10. Composition according to one of the preceding claims, characterized in that it is presented in the form of a nail-care product.

11. Use of pyrogenic silica as thickening agent for a composition according to one of Claims 1 to 10.

## Patentansprüche

1. Zusammensetzung zum Auftragen auf die Nägel, die in durchscheinender Form vorliegt und mindestens ein filmbildendes Mittel, mindestens einen Weichmacher und mindestens ein Lösungsmittel enthält, dadurch gekennzeichnet, daß sie ferner pyrogene Kieselsäure enthält.

2. Zusammensetzung nach Anspruch 1, dadurch gekennzeichnet, daß die Kieselsäure hydrophile oder hydrophobe pyrogene Kieselsäure ist.

3. Zusammensetzung nach einem der vorhergehenden Ansprüche, dadurch gekennzeichnet, daß sie ferner mindestens einen Bestandteil in Form von Partikeln und/oder in Form von Fasern enthält.

4. Zusammensetzung nach Anspruch 3, dadurch gekennzeichnet, daß der Bestandteil unter Nylonfasern, Aramidfasern, Diamantpartikeln, Bornitridpartikeln, Perlglanzpartikeln und/oder Bimssteinpartikeln ausgewählt ist.

5. Zusammensetzung nach einem der vorhergehenden Ansprüche, dadurch gekennzeichnet, daß sie ferner mindestens einen kosmetischen Wirkstoff enthält.

6. Zusammensetzung nach Anspruch 5, dadurch gekennzeichnet, daß der kosmetische Wirkstoff unter Hydratisierungsmitteln, Härtungsmitteln, Sonnenschutzfiltern, Vitaminen, Verdickungsmitteln und/oder Materialien biologischen Ursprungs ausgewählt ist.

7. Zusammensetzung nach einem der vorhergehenden Ansprüche, dadurch gekennzeichnet, daß die Kieselsäure in einem Mengenanteil von 0,1 bis 3 Gew.-% und vorzugsweise 0,4 bis 0,8 Gew.-%, bezogen auf das Gesamtgewicht der Zusammensetzung, vorliegt.

8. Zusammensetzung nach einem der vorhergehenden Ansprüche, dadurch gekennzeichnet, daß sie eine optische Dichte von weniger als 1 und vorzugsweise eine optische Dichte im Bereich von 0,1 bis 0,6 aufweist.

9. Zusammensetzung nach einem der vorhergehenden Ansprüche, dadurch gekennzeichnet, daß sie in Form eines Nagellacks vorliegt.

10. Zusammensetzung nach einem der vorhergehenden Ansprüche, dadurch gekennzeichnet, daß sie in Form eines Produkts zur Nagelpflege vorliegt.

11. Verwendung von pyrogener Kieselsäure als Verdickungsmittel in Zusammensetzungen nach einem der Ansprüche 1 bis 10.
